# EUROPEAN PATENT SPECIFICATION

(11) **EP 4 501 396 B1**
(45) Date of publication and mention of the grant of the patent: **10.09.2025**
(21) Application number: 23382805.2
(22) Date of filing: 01.08.2023
(51) Int. Cl.: A61N 1/36, A61N 1/04, H04R 1/10, H04R 25/00

(54) **DEVICE FOR TREATING INNER EAR DISORDERS SUCH AS TINNITUS OR HEARING LOSS**
VORRICHTUNG ZUR BEHANDLUNG VON INNENOHRERKRANKUNGEN WIE TINNITUS ODER GEHÖRVERLUST
DISPOSITIF POUR TRAITER DES TROUBLES DE L'OREILLE INTERNE TELS QUE L'ACOUPHÈNE OU LA PERTE AUDITIVE

(43) Date of publication of application: 05.02.2025
(73) Proprietor: JTG Solutions, S.L., 08007 Barcelona (ES)
(72) Inventor: MARTÍNEZ-MONCHE ZARAGOZA, Gonzalo, 08007 Barcelona (ES)
(74) Representative: Sugrañes, S.L.P.

(56) References cited:
- US-A1- 2017 353 807
- US-A1- 2019 313 184
- US-A1- 2020 038 658
- US-A1- 2022 086 574

## Description

### Technical field of the invention

The device for treating inner ear disorders of the present invention is of the type that enables electrical stimuli to be generated that are suitable for treating auditory disorders such as tinnitus or hearing loss.

### Background of the invention

Hearing loss is classified as conductive or sensorineural. Conductive hearing loss tends to have a mechanical origin since the sound is not conducted effectively from the outer ear to the middle ear. Discrimination is barely affected, and most cases can be resolved by means of surgery or a correct prescriptive amplification.

Sensorineural hearing loss as a hearing disorder tends to appear when there is damage to the inner ear, or in the nerve pathway between the inner ear and the brain. This is the most common type of hearing loss, and it cannot be corrected medically or through surgery. The majority of cases can be resolved by a correct prescriptive amplification.

The level of hearing loss of a patient can be measured in a known manner by means of an audiogram. An audiogram is a graph that shows the results of pure tone audiometry tests, automatic audiometry or computerised audiometry, normally in a standardized frequency set. It illustrates the type, grade and configuration of the hearing loss.

Another sensorineural hearing disorder is tinnitus, which presents as a continuous tone that is perceived by a patient in one or both ears.

In order to determine the frequency of the tinnitus, a broad band noise is introduced in a known manner and the patient is asked to identify when their tinnitus is no longer audible. Other additional tinnitus tests include residual inhibition, which registers the amount of time the sound of an individual in the ears is reduced or disappears after a period of masking.

Devices are known for the treatment of sensorineural hearing disorders such as tinnitus by means of an electrical stimulation signal. However, the electrical stimulation signal in the known devices is designed to generate an electrical current that stimulates neurons and maintains a neuronal activation rate, it being necessary to maintain a frequency that enables the neurons to recover from the stimulation. This entails using low frequencies to maintain the neuronal activation rate.

The location where the electrical stimulation signal is applied in a patient is also important as it is necessary to apply said electrical stimulation signal to points adjacent to nerves, the placement of the devices that apply the electrical stimulation signal being important in order to achieve satisfactory results.

Devices are known in which the electrical stimulation signal is applied on the tongue. These devices are provided with a peripheral that the patient must insert in the mouth to be in contact with the tongue in order to perform a therapeutic effect. The patent document EP2842530 discloses one of these devices. However, the use of these devices is uncomfortable for the patient. Another exemplary device for tinnitus treatment is disclosed in US 2017/353807 A1.

Therefore, an object of the present invention is to disclose a device for treating hearing disorders that is simple, easy for the patient to use and effective.

### Description of the invention

The invention provides a system according to claim 1. The device comprised in the system of the present invention for treating inner ear disorders such as sensorineural disorders of the inner ear such as tinnitus or hearing loss in a patient is of the type that comprises at least one electrode adapted to transmit an electrical stimulation signal to the patient.

In essence, the device is characterised in that the at least one electrode is mounted on a support configured such that when the at least one electrode is placed on a patient it is arranged on the anterosuperior part of the mastoid process of the patient, behind the insertion line of the auricle, on the skin, such that the electrical stimulation signal transmitted from the at least one electrode reaches the anterosuperior part of the mastoid process of the patient. Naturally, contact with the hair must be minimised such that the one or more electrodes are arranged on the skin on the extension of the imaginary horizontal line that passes through the external auditory canal. Given that this part of the mastoid process on which the one or more electrodes will be placed is located behind the auricle, it is important that the device enable the one or more electrodes to be arranged on the skin behind the auricle and on the anterior end of the mastoid process that is next to the external auditory canal. The one or more electrodes are thus arranged away from the muscle insertion of the sternocleidomastoid. It is intended that the device has two electrodes to conduct the electrical stimulation signal therebetween, although it is only necessary for one of the electrodes to be arranged on the anterosuperior part of the mastoid process, it being possible for the other electrode to be arranged on another point of the head. However, when the two electrodes are mounted on the support and are arranged on the anterosuperior part of the mastoid process, the energy of the electrical stimulation signal is concentrated on the anterosuperior part of the mastoid process, thus achieving a better effect. Naturally, the electrical stimulation signal, which is alternating and does not have a direct component, is carried through the skin of the patient between the electrodes, spreading the electrical stimulation signal through the skin to the indicated part of the mastoid process.

The electrical stimulation signal has a main frequency component greater than 200 kHz and lower than 600 kHz, preferably greater than 300 kHz, the main frequency of the electrical stimulation signal being within the intermediate frequency band according to the recommendation ITU-R V.431-8 of 8 August 2015, for example within the lower 10% of this intermediate frequency band, between 300 and 600 kHz. Advantageously, by means of the device object of the present invention, the electrical stimulation signal is applied by means of the electrodes, stimulating the anterosuperior part of the mastoid process of the patient, target, which has a spongy constitution with multiple cavities and is adjacent to the external auditory canal, producing a localised diathermic effect that achieves the therapeutic effect of improving inner ear disorders. A main frequency component can be a frequency peak or lobe that includes this main frequency, with a certain band width, or a frequency band that includes the main frequency. This main frequency component can also be the fundamental frequency of a periodic signal, such as, for example, a square, triangular or sinusoidal signal. It is intended that a main frequency component is preferably greater than one of the following frequencies: 200, 210, 220, 230, 240, 250, 260, 270, 280, 290, 300, 310, 320, 330, 340, 350, 360, 370, 380, 390, 400, 410, 420, 430, 440 or 450 kHz; and is preferably lower than one of the following frequencies: 600, 590, 580, 570, 560, 550 o 540 kHz. Naturally, other electrical stimulation signals that have sufficient power within this frequency band, for example, greater than 5 W, or greater than 6 W, or greater than 7 W, such as 7.5 W, can be considered to have main frequency component in this band and can be used for the therapeutic effect, for example upon being applied by means of the device of the present invention. Naturally, it is advisable that the power of the electrical stimulation signal be concentrated within the frequency bands that produce the therapeutic effect in order to optimise the power concentration and use of the signal.

In an embodiment variant, the electrical stimulation signal has a main frequency component between 450 and 540 kHz, within the intermediate frequency band. Specifically, the electrical stimulation signal can have a main frequency component of 495 kHz, which although ideally has a band width that is as narrow as possible, it is contemplated that it can have a band width up to 90 kHz, thus encompassing the frequencies between 450 and 540 kHz, in which an optimal diathermic effect is achieved in order to improve the inner ear disorders. As such, a main frequency component is preferably greater than 450, 460, 470, 480 or 490 kHz, and is preferably less than 540, 530, 520, 510 or 500 kHz. It is envisaged that the electrical stimulation signal has a voltage of 5 V and a current of 1.5 A, it even being possible to generate it by means of a portable battery that could be incorporated into the device.

In an embodiment variant, in the direction with the smallest distance between the first electrode and the second electrode, the distance between the first electrode and the second electrode is less than the distance between the ends of the first electrode and the distance between the ends of the second electrode, thus promoting that when they are applied to the skin of the patient, the resistance of the skin between the first electrode and the second electrode is low and enables the passage of the electrical stimulation signal and its propagation towards the anterosuperior part of the mastoid process. Specifically, it is provided that the separation distance is between 3 and 7 millimetres, typically 5 millimetres, the distance between the ends of each electrode being between 10 and 15 millimetres, typically 14 millimetres, the electrodes being 10 millimetres wide, these dimensions being suitable for the electrodes to be arranged on the anterosuperior part of the mastoid process. Naturally, if the support only has one electrode, the other electrode of the pair of electrodes is arranged in another area of the head, the distance between the ends could be up to 35 millimetres. However, being able to use different pairs of electrodes to treat each inner ear of the patient is intended.

In an embodiment variant, the first electrode and the second electrode are equal, which prevents decompensations and an electrode from heating or wearing more than the other.

In an embodiment variant, the first electrode and the second electrode are placed in a support that enables a fast and easy placement of the electrodes on the mastoid process of the patient, which can be performed even by the patient him/herself without the help of a healthcare professionals. It is intended that the support can have different forms suitable such that when it is placed on the head of the patient the one or more electrodes are correctly positioned. Naturally, it is intended that the support can have different forms that easily enable the correct placement of the one or more electrodes on the patient. These shapes can be, among others, in the form of earphones, a hearing aid, headphones, hook earbuds, in the form of glasses or glasses temples or in the form of a helmet or hat, such that when the support is placed on the patient, the one or more electrodes that transmit the electrical stimulation signal are correctly arranged on the anterosuperior part of the mastoid process of the ear to be treated.

In an embodiment variant, the first electrode and the second electrode are arranged symmetrically on the support, thus achieving that the electrical stimulation signal flows homogeneously.

In an embodiment variant, the first electrode and the second electrode are arched, enabling the possibility of adapting to the shape of the head of the patient and even favouring the depression thereof into the skin of the head of the patient. In this way, it is also intended that the support is adapted to exercise a certain force in the direction normal to the electrodes, improving the contact thereof with the skin, for example when the support is in the form of headphones. Naturally, it is intended that only one electrode can be arched and the other can be arranged on different parts of the patient, although it is preferably contemplated that both are arched and are located close to each other, on the anterosuperior part of the mastoid process.

In an embodiment variant, the first electrode and the second electrode are parallel to each other, thus achieving that the electrical stimulation signal flows homogeneously.

In an embodiment variant, the support is adapted to be placed on the head of the patient, the support being configured such that, when the support is placed on the head of the patient, the mastoid process of the patient is arranged adjacent to the first electrode and the second electrode.

In an embodiment variant, the support comprises an earcup adapted to be placed on the auricle of the patient. Naturally, it is intended that the support can have two earcups, each earcup being provided with a first electrode and a second electrode, each pair of electrodes being adapted to be arranged adjacent, respectively, to each one of the two mastoid processes on each side of the head of the patient, even being able to use the same generation means and the same electrical stimulation signal, which can form part of the same device or be arranged remotely, for example in a console connected to the device, for example by means of a cable.

In an embodiment variant, the first electrode and the second electrode are arranged in the periphery of the earcup, thus facilitating the correct arrangement of the first electrode and the second electrode when the earcup is placed and that the patient can clearly feel the first electrode and the second electrode.

In an embodiment variant, the support comprises an earcup the cross section of which, for example, the horizontal cross section thereof, determines a horseshoe arch, that is it is C-shaped, forming an arch greater than 180 degrees that determines an inner area where the auricle is housed, being trapped. Therefore, the earcup is adapted to be placed covering and surrounding the auricle of the patient, at least one electrode being arranged at one end of the earcup, such that the electrode is arranged behind the auricle of the patient, on the anterosuperior part of the mastoid process when the patient places the earcup, which may be mounted on earphones.

In an embodiment variant, the support is also provided with sound emitting means, such as a speaker, which complements the therapeutic effect of the electrical stimulation signal on the mastoid process.

In an embodiment variant, the sound emitting means are adapted to emit sound with a stop frequency band, the stop frequency band being the frequency band of the tinnitus of the patient.

In an embodiment variant, the device comprises processing means adapted to stop a frequency band of an audio signal, such that the stopping of the frequency band by means of signal processing techniques can be programmed, for example in a processor.

In an embodiment variant, the support are earbuds that enable the one or more electrodes to be duly placed on the target part of the mastoid process of the patient.

Therefore, it is possible to perform through the use, for example, of a device of the present invention a therapeutic method for treating inner ear disorders, for example sensorineural inner ear disorders such as tinnitus or hearing loss. As such ear disorders can be treated by applying an electrical stimulation signal to the anterosuperior part of the mastoid process, by means of electrodes, for example a stimulation signal as described above, for example with a main frequency component greater than 200 and 600 kHz, more specifically an intermediate frequency in this range, more specifically an electrical stimulation signal with a main frequency component between 450 and 540 kHz and more specifically an electrical stimulation signal with a main frequency component of 495 kHz.

### Brief description of the drawings

In order to complement the description made herein and with the aim of improving the understanding of the features of the invention, a set of drawings is attached to the present specification in which the following has been represented in an illustrative and non-limiting manner:
Figure 1 shows a diagram of a device of the present invention;
Figure 2 shows an example of an electrical stimulation signal for a device of the present invention;
Figure 3 shows, on the skull of a patient, the arrangement of the anterosuperior part of the mastoid process, which is the target for a device of the present invention.
Figure 4 shows an operation diagram of a device of the present invention on a patient.
Figure 5 shows a first electrode and a second electrode for a device of the present invention in a correlative fitting position.
Figures 6a and 6b show a first embodiment of a device according to the present invention placed on the head de a patient;
Figures 7a and 7b show the device of figures 6a and 6b;
Figure 7c shows a detailed view of the cross section of the device of Figures 7a and 7b;
Figures 8a and 8b show a second embodiment of a device according to the present invention placed on the head of a patient; and
Figure 9 shows a third embodiment of a device according to the present invention placed on the head of a patient.

### Detailed description of the drawings

The device 1 of the system according to the invention has been used to perform a pilot clinical study to assess the effect of applying high frequencies on the mastoid process 101 of adult patients with tinnitus, and also sensorineural hearing loss.

The device 1 used further comprises generation means 2 for generating an electrical stimulation signal 3 and electrodes 5a, 5b to apply the electrical stimulation signal 3 on the patient 100. In order to facilitate the correct placement of the electrodes 5a, 5b on the mastoid process 101 of the patient 100, which is the target zone, the electrodes 5a, 5b are configured to, upon being placed on the head 102 of the patient 100, be correctly placed in order to be able to apply the electrical stimulation signal 3 on the anterosuperior part of the mastoid process 101 of the patient 100.

In an embodiment variant, the electrical stimulation signal 3 is a high frequency signal, which has a main frequency component greater than 200 kHz and less than 600 kHz. This frequency range is advised against in the state of the art for treating hearing disorders, as it is higher than the neural activation rate, since the known devices act directly on the nerves, stimulating them to treat hearing disorders, such as tinnitus.

However, applying a frequency greater than 200 kHz and less than 600 kHz on the mastoid process 101, by applying an electrical stimulation signal 3 of 5 V and 1.5 A, has been surprisingly observed to have a very localised diathermic effect that has, after the study, not only allowed an improvement to be obtained in the tinnitus previously diagnosed in patients, but also an improvement in sensorineural hearing loss. Naturally, it is expected that other voltages and amperages have the same effect.

The inclusion criteria of the study were patients between 18 and 70 years of age, with no debilitating chronic illnesses, no drug treatment, with indifferent hearing loss, no vertigo or instability. In addition, tinnitus evolving for more than 6 months, which is non-pulsatile, with gradual onset, not linked to traumatic events, with a tinnitus volume scale (1-100) of between 40 and 60, with a tinnitus handicap inventory (THI) of between 25 and 65. An experimental group, with experimental treatment, and a placebo group are established, with one group or another being assigned randomly.

During the study, a screening phase was initially performed, which lasted a maximum of 3 months which was necessary to recruit the participants of the study. It is expected that the patients that are already being treated in USD Audiology, subjected to a hearing test and audiometric tests in the last six months

The treatment performed in the study lasted for a total of 6 consecutive weeks, with 2 sessions a week lasting approximately 40 minutes each, using the device 1 for about 15-20 min per ear, to be performed with a separation between sessions of min. 2 and max. 4 days one from another.

The end of the treatment has been set at the end of the sixth week. Subsequently, two control assessments have been planned, one of which takes place 12 weeks and another 6 months after the start of the study.

The results of the study show a significant improvement of both THI (>10 points) and a minimum level of masking (>=5dB) at the end of the treatment in the experimental group compared to the placebo group. In addition, although the study was initially designed to particularly research tinnitus, according to the study, a large number of patients were found to have improved PTA of more than 10dB HL in the treated ear upon finishing the treatment and the following three months.

According to the study, these results can be related to a diathermic effect located in the metabolism of the inner ear and homeostasis, meaning that it is expected that they can also be applied to other inner ear disorders, for example to treat patients with Ménière's disease.

This study has been performed using a device 1 according to the present invention, embodiments of which are described below. Naturally, it is intended that other embodiments of the device 1, the embodiments of which are not described, would also provide the desired therapeutic effect.

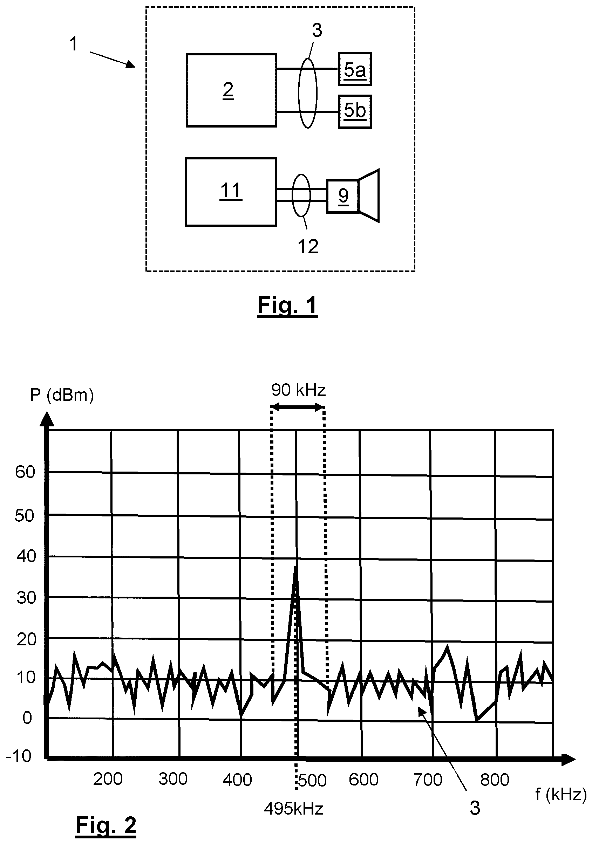

Figure 1 shows a diagram of the device 1 for treating sensorineural disorders of the inner ear such as tinnitus or hearing loss in a patient 100. As can be seen, the represented device 1 comprises generation means 2 for generating an electrical stimulation signal 3 and electrodes 5a, 5b of the electrical stimulation signal 3 to the patient 100, specifically, the electrodes 5a, 5b are configured to apply the electrical stimulation signal 3 on the anterosuperior part of the mastoid process 101 of the patient 100. The device 1 comprises a first electrode 5a and a second electrode 5b. The placement of at least one electrode 5a, 5b on the patient 100 is important for the invention, as shall be detailed below. Naturally, the generation means 2 can be provided outside the device 1, for example in a console, peripheral or remote control, and transfer the electrical stimulation signal 3 for example by means of a cable directly to the electrodes 5a, 5b of the device 1.

In the diagram shown, the device 1 is further provided with sound emitting means 9 adapted to emit sound with a stop frequency band, having processing means 11 adapted to stop a frequency band of an audio signal 12. These sound emitting means 9 are optional and the incorporation thereof has been shown to improve still more the therapeutic effect of the applied electrical stimulation signal 3. Naturally, the processing means can also be provided outside the device 1, for example a console, peripheral or remote control, and transfer the audio signal 12 for example, by means of a cable directly to the sound emitting means 9 of the device 1. It is intended that a single cable can be used for one or more of supplying the device 1, transferring the audio signal 12, optionally already processed, and sending other data, such as start, stop commands or commands to transfer records of the status of the device 1.

Figure 2 shows an electrical stimulation signal 3 suitable for use in the device 1 of the present invention and used in the study. It can be seen that figure 2 shows the frequency spectrum of an electrical stimulation signal 3 suitable for use in a device 1 of the present invention, specifically the power spectrum or power spectral density, where it can be seen that the electrical stimulation signal 3 has a main frequency component greater than 200 and less than 600 kHz, specifically the main frequency is between 450kHz and 540kHz with its peak around 495kHz. It is possible for the signal to additionally have other main frequency components, for example, frequency peaks in other ranges. It can be seen that in the represented electrical stimulation signal 3. It can be seen in the represented electrical stimulation signal 3 that is of the band width of the main frequency, the other frequency components of the signal are sufficiently attenuated, being considered noise. Thus, it is intended that the electrical stimulation signal 3 can be an essentially sinusoidal signal with a fundamental frequency around 495kHz, although other forms of electrical stimulation signal 3 are also considered, the main or fundamental frequency of which is the same, for example a square signal, or saw-tooth signal, which have other frequency components, but maintain the fundamental frequency within the previously indicated range. It can be seen that the electrical stimulation signal 3 is represented in dBm, meaning that the scale is logarithmic, the power of the peak being much greater than 495kHz, which would be slightly less than 40 dBm, while the power of the rest of the frequencies would be much lower. It can be seen that given that in this case the electrical stimulation signal 3 is a 5 V and 1.5 A signal, its power would be 7.5 W, corresponding to 38.75 dBm which is approximately the value of the peak at 495 kHz, meaning that primarily all the power of the signal is around this frequency, specifically the power is concentrated in this peak frequency and possibly within a band width up to 90 kHz, between 450kHz and 540kHz, where the power falls 30 dBm. Outside this band width, in the signal 3 shown, the power of the signa is very low, comparable to noise. The possibility of using another type of electrical stimulation signal 3, with other frequencies, is not ruled out in order to obtain the therapeutic effect when applied to the anterosuperior part of the mastoid process 101, specifically other signals with main frequencies within the intermediate band, or frequencies close to this band, such as between 200 kHz and 600 kHz, not ruling out that effects can be produced even with lower frequencies and that, for example, are greater than 100 kHz.

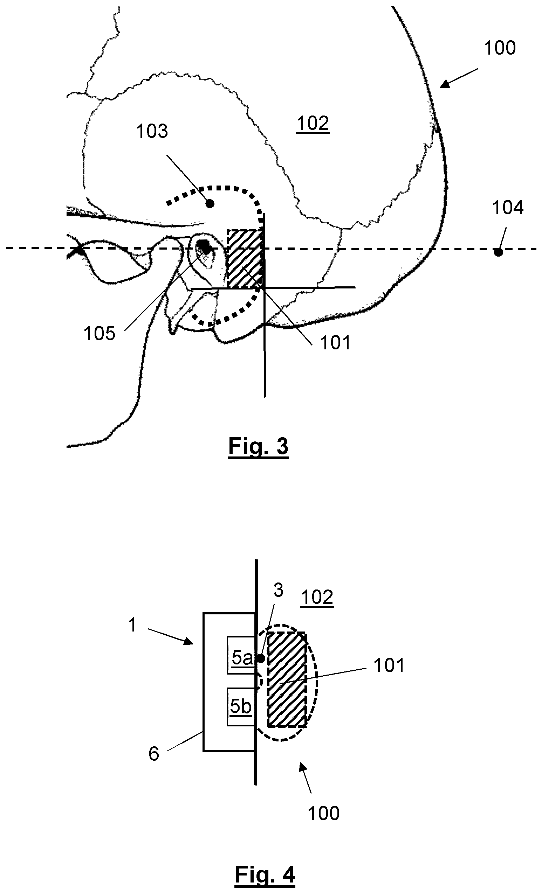

Figure 3 shows the anterosuperior part of the mastoid process 101 of a patient 100 on which the at least one electrode 5a, 5b of a device 1 of the present invention must be placed, this anterosuperior part is the target part on which the electrical stimulation signal 3 must impact as it circulates between the two electrodes 5a, 5b, such that the electrical stimulation signal 3 transmitted from the at least one electrode 5a, 5b reaches the anterosuperior part of the mastoid process 101 of the patient 100. The electrical stimulation signal 3 that is transmitted from the at least one electrode 5a, 5b reaches the anterosuperior part of the mastoid process 101 of the patient 100 when at least one electrode 5a, 5b is arranged on the anterosuperior part of the mastoid process 101. Figure 3 shows the auricle 103 of the patient 100, showing that the anterosuperior part of the mastoid process 101 is covered by the auricle 103, such that the device 1 must be configured to access the posterior part of the auricle 103 in order to arrange the one or more electrodes 5a, 5b on the anterosuperior part of the target mastoid process 101, such that the passage of the electrical stimulation signal 3 stimulates this target zone. This target zone is the middle part of the mastoid process behind the insertion line of the auricle 103 on the skin, which may be considered the anterosuperior quadrant of the mastoid process 101, in the zone adjacent to the external auditory canal 105, typically in the continuation of the horizontal imaginary line 104 that passes through the external auditory canal. Thus, as it is placed on the patient 100, the at least one electrode 5a, 5b is arranged on the anterosuperior part of the mastoid process 101 of the patient 100 such that the electrical stimulation signal 3 transmitted from the at least one electrode 5a, 5b reaches the anterosuperior part of the mastoid process 101 of the patient 100, stimulating it to obtain a therapeutic effect.

Figure 4 shows a diagram of the electrodes 5a, 5b to transmit the electrical stimulation signal 3 of the device 1 when they are applied to a patient 100. It can be seen that the electrodes 5a, 5b comprise a first electrode 5a and a second electrode 5b that are arranged on the head 102 of the patient 100. The electrodes 5a, 5b are mounted on a support 6 that facilitates the application of the electrodes 5a, 5b on the anterosuperior part of the mastoid process 101 which is where the electrical stimulation signal 3 is to be applied. As can be seen, the electrical stimulation signal 3 is conducted through the patient 100 between the electrodes 5a, 5b propagating the electrical stimulation signal 3 by covering the mastoid process 101 of the patient 100. It is intended depending on the resistance of the patient 100 between the electrodes 5a, 5b that the electrical stimulation signal 3 can also be radiated, for example partially, since the connection between the electrodes 5a, 5b through the patient 100 can be modelled as a resistance and condenser in parallel.

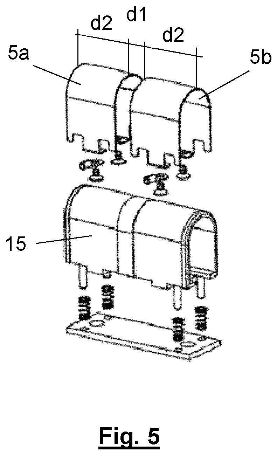

Figure 5 shows an example of electrodes 5a, 5b for a device 1 of the present invention. It can be seen that the electrodes 5a, 5b are arched, to facilitate the contact with the skin of the patient 100. It can also be seen that in the direction of the smallest distance between the first electrode 5a and the second electrode 5b, the separation distance d1 between the first electrode 5a and the second electrode 5b is less than the distance between ends d2 of the first electrode 5a and the distance between ends d2 of the second electrode 5b. In this case the distance between ends d2 of each electrode 5a, 5b is the same because the electrodes 5a, 5b are equal and parallel to each other. It can be seen that in this case the electrodes 5a, 5b that are made of metal, are attached to a dielectric base 15 that is installed in the support 6 of the device 1, such that the electrodes 5a, 5b are arranged on the support 6.

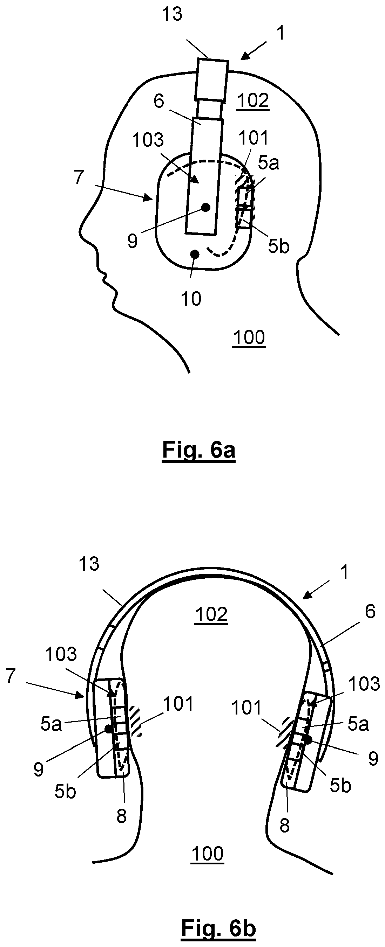

Figures 6a and 6b show a first embodiment of the device 1 according to the invention, which comprises a first electrode 5a and a second electrode 5b. It can be seen that the first electrode 5a and the second electrode 5b are equal and parallel to each other, and that in the direction of the smallest distance between the first electrode 5a and the second electrode 5b, the distance between the first electrode 5a and the secondo electrode 5b is less than the distance between ends of the first electrode 5a and the distance between ends of the second electrode 5b and are arranged in an earcup 10 provided with a pad 8.

The first electrode 5a and the second electrode 5b are placed in a support 6 which in this case are in the form of earphones 7, also known as headphones or headsets, that facilitate the placement of the device 1 on the head 102 of a patient 100, such that the electrodes 5a, 5b are configured arranged to apply the electrical stimulation signal 3 on the anterosuperior part of the mastoid process 101 of the patient 100, that is, they are duly placed on the skin of the head 102 of the patient 100, behind the auricle 103. The electrodes 5a, 5b apply, during the treatment of the inner ear disorder, an electrical stimulation signal 3 as previously described, with a main frequency component greater than 200 kHz and less than 600 kHz, preferably a signal with a main frequency within an intermediate frequency band.

It is intended that the device 1 is provided with a timer to limit the time during which the electrical stimulation signal 3 can be applied, preferably enabling a use of 15 or 20 minutes for each ear, and not enabling the electrical stimulation signal 3 to be applied again in an ear until at least 24 hours have passed.

It can be seen that the support 6 comprises an earcup 10 adapted to be placed on and around the auricle 103 of the patient 100 with a pad 8 for a more comfortable fastening. El first electrode 5a and the second electrode 5b are arranged on the periphery of the earcup 10, which enables the electrodes 5a, 5b to be easily placed behind the auricle 103, on the anterosuperior part of the mastoid process 101 of the patient 100. Given that the device 1 has two earcups 10, it is possible to have pairs of electrodes 5a, 5b in each one of the earcups 10. Naturally, the treatment of the inner ear disorders is only performed in the ear affected by, for example, tinnitus or hearing loss. This treatment is performed by applying the electrical stimulation signal 3 by means of electrodes 5a, 5b and optionally for treating tinnitus by emitting sound by means of the sound emitting means 9 with a stop frequency band, the stop frequency band being that which corresponds to the frequency of the tinnitus of the patient 100 in that ear, which has been previously determined via known techniques.

In this case, given that the support 6 of are headphones 7, sound emitting means 9 can also be used, incorporated in the earcups 10 to emit sound to complement the treatment of the inner ear disorders as described above. Naturally, it is intended that the headphones 7 are provided with an adjustable headband 13 that joins the two earcups 10 above the head 102 of the patient 100, such that the patient 100 may adjust the device 1 such that they are correctly arranged surrounding and clasping the auricles 103, the electrodes 5a, 5b thus automatically being correctly placed on each one of the mastoid processes 101, exerting a certain pressure on the head 102 to ensure the contact of the electrodes 5a, 5b. Naturally, the earcups 10 may be devoid of the headband 13, for example being fitted on the auricle 103 of the patient, or provided with other fastening means, which enable the correct arrangement thereof.

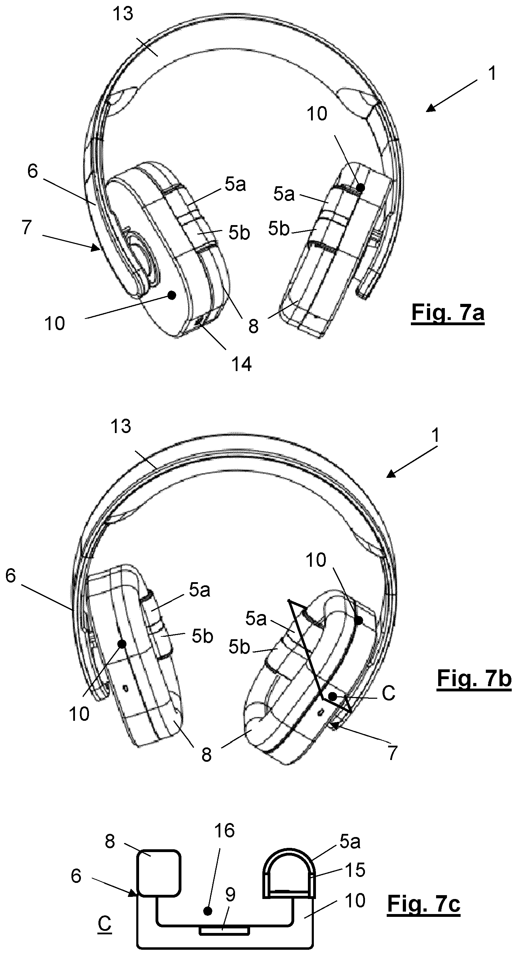

Figures 7a and 7b show more detailed views of this first embodiment of the device 1 in the form of headphones 7 wherein the support 6 comprises one earcup 10 adapted to be placed on the auricle 103 of the patient 100, the first electrode 5a and the second electrode 5b being arranged symmetrically, arched at the periphery of the earcup 10, and parallel to each other. It can also be seen that in order for the device 1 to be self-supporting, the power supply means, such as batteries, are arranged inside the earcups 10, the earcups 10 being provided with a charging port 14, for example a USB port suitable for connecting a power cable and sending data. It is also intended that the device 1 can be connected directly to a power source, in this case being provided with a power cable. When the device 1 is self-supporting it allows greater freedom of movement for the patient 100 during the use thereof, but it is also intended that it can be connected to a power source, for example by means of a cable. It is intended that the cable can directly connect the electrodes 5a, 5b to generation means 2 of the electrical stimulation signal 3 arranged separated, for example arranged in a control console connected to the headphones 7. In this case, the device 1 is formed by the headphones and the control console. This console can also provide the sound already duly processed directly to the sound emitting means 9. It is also intended that when the device 1 is self-supporting and the sound emitting means 9 are used, it is provided with receiving means of a wireless radio signal that enables a wireless radio signal to be received from remote emitters, which wireless radio signal is converted into the audio signal 12 to be emitted by the sound emitting means 9. These receiving means of a Wireless radio signal can be a Bluetooth sound receiver or even a modulated frequency radio receiver. In this case, it is intended that the audio signal 12 can be sent having already processed, for example with the stop frequency band, or can be processed after it has been received. It is also intended that when the device 1 is self-supporting and contained, the processing means 11 of the audio signal 12 generate a predetermined audio signal 12, for example a white noise signal with a stop frequency band. It is intended that the device 1 has control means that enables the indication of the frequency to be stopped.

Figure 7c shows the cross section C that can be seen in Figure 7b to illustrate that the support 6 comprises an earcup 10 with a cross section C that determines a horseshoe arch, that is C-shaped, forming an arch greater than 180 degrees to thus form an inner area 16, for example 5 cm by 7 cm, with an edge having an overhang of at least 1 cm, in which to house, the auricle 103, trapping it. Therefore, the earcup 10 is adapted to be placed covering and surrounding the auricle 103, with, in this case, the first electrode 5a arranged at one end of the earcup 10, such that the first electrode 5a, which is mounted on the base 15 is arranged behind the auricle 103 of the patient 100, on the anterosuperior part of the mastoid process 101. A flexible pad 8 is also included at the end of the earcup 10 to facilitate the housing of the auricle 103 in the inner area 16. Thus, when the patient places the earcup 10 on themselves, which may be mounted on headphones 7, the electrodes 5a, 5b are duly placed for the treatment without the involvement of medical personnel being necessary, which means that the patient 100 can perform the treatment themselves, for example at home. Naturally, the earcup 10 can have a single electrode 5a, 5b, the other electrode being arranged in the other area of the body of the patient 100, although for a more effective treatment, it is recommended that both electrodes 5a, 5b be arranged on the anterosuperior part of the mastoid process 101, thus minimizing contact with the hair. It can also be seen that the ear 10 also has sound emitting means 9, a loudspeaker, that can be used to complement the treatment.

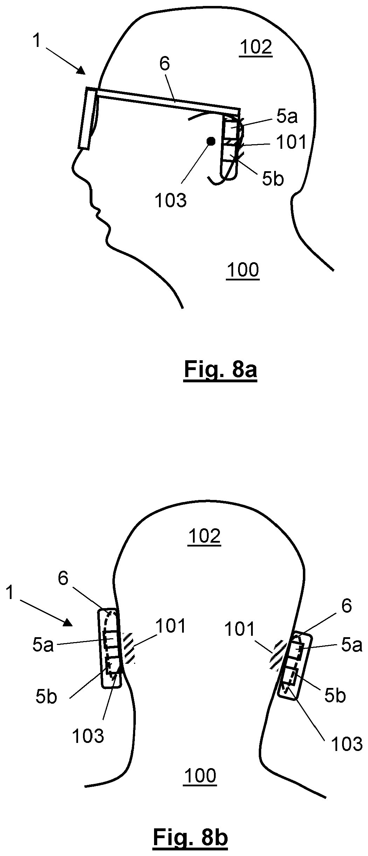

Figures 8a and 8b show a second embodiment of the device 1 according to the invention, wherein the electrodes 5a, 5b are mounted on a support 6 in the form of a glasses frame, the one or more electrodes 5a, 5b being mounted on the curved ends of the temples, such that they are arranged behind the auricle 103 and on the anterosuperior part of the mastoid process 101.

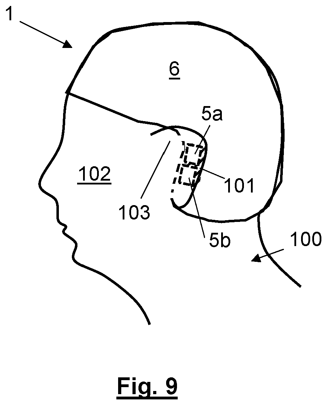

Figure 9 shows a third embodiment of the device 1 according to the invention, wherein the electrodes 5a, 5b are mounted on a support 6 in the form of a helmet or hat, the helmet or hat being adapted to be arranged behind the auricle 103, the part of the helmet or hat adapted to be arranged behind the auricle 103 being provided with the one or more electrodes 5a, 5b, such that they are arranged behind the auricle 103 and on the anterosuperior part of the mastoid process 101.

## Claims

1. A system comprising:
- a device (1) for treating inner ear disorders such as tinnitus or hearing loss in a patient (100) comprising at least one electrode (5a, 5b) adapted to transmit an electrical stimulation signal (3) to the patient (100), wherein the at least one electrode (5a, 5b) is mounted on a support (6) configured such that when it is placed on the patient (100) the at least one electrode (5a, 5b) is arranged on the anterosuperior part of the mastoid process (101) of the patient (100) such that the electrical stimulation signal (3) transmitted from the at least one electrode (5a, 5b) reaches the anterosuperior part of the mastoid process (101) of the patient (100), and
- generation means (2) connected to the at least one electrode (5a, 5b), the generation means (2) configured to provide the electrical stimulation signal (3) to the at least one electrode (5a, 5b),
**characterised in that** the electrical stimulation signal (3) has a main frequency component greater than 200 kHz and less than 600 kHz.

2. The system according to the preceding claim, **characterised in that** the electrical stimulation signal (3) has a main frequency component of between 450 kHz and 540 kHz.

3. The system according to the preceding claim, **characterised in that** the electrical stimulation signal (3) has a main frequency component of 495 kHz.

4. The system according to any one of the preceding claims, **characterised in that** it comprises a first electrode (5a) and a second electrode (5b) mounted on the support (6) configured such that when the electrodes (5a, 5b) are placed on the patient (100) they are arranged on the anterosuperior part of the mastoid process (101) of the patient (100).

5. The system according to the preceding claim, **characterised in that**, in the direction of the smallest distance between the first electrode (5a) and the second electrode (5b), the separation distance (d1) between the first electrode (5a) and the second electrode (5b) is less than the distance between ends (d2) of the first electrode (5a) and the distance between ends (d2) of the second electrode (5b).

6. The system according to any one of claims 4 to 5, **characterised in that** the first electrode (5a) and the second electrode (5b) are equal.

7. The system according to any one of claims 4 to 6, **characterised in that** the first electrode (5a) and the second electrode (5b) are arranged symmetrically on the support (6).

8. The system according to any one of claims 4 to 7, **characterised in that** the first electrode (5a) and the second electrode (5b) are arched.

9. The system according to any one of claims 4 to 8, **characterised in that** the first electrode (5a) and the second electrode (5b) are parallel to each other.

10. The system according to any one of the preceding claims, **characterised in that** the support (6) comprises an earcup (10) the cross section (C) of which determines a horseshoe arch, the earcup (10) being adapted to be placed covering and surrounding the auricle (103) of the patient (100), the at least one electrode (5a, 5b) being arranged at one end of the earcup (10).

11. The system according to any one of the preceding claims, **characterised in that** it is also provided with sound emitting means (9).

12. The system according to the preceding claim, **characterised in that** the sound emitting means (9) are adapted to emit sound with a stop frequency band.

13. The system according to the preceding claim, **characterised in that** it comprises processing means (11) adapted to stop a frequency band of an audio signal (12).

14. The system according to any one of the preceding claims, **characterised in that** the support (6) are headphones (7).

## Patentansprüche

1. System umfassend:
- eine Vorrichtung (1) zur Behandlung von Innenohrerkrankungen wie Tinnitus oder Gehörverlust in einem Patienten (100), welche mindestens eine Elektrode (5a, 5b) umfasst, welche dazu angepasst ist, dem Patienten (100) ein elektrisches Stimulationssignal (3) zu übertragen, wobei die mindestens eine Elektrode (5a, 5b) auf einem Träger (6) montiert ist, welche so ausgebildet ist, dass, wenn auf dem Patienten (100) platziert wird, die mindestens eine Elektrode (5a, 5b) auf dem anterosuperioren Teil des Warzenfortsatzes (101) des Patienten (100) angeordnet ist, sodass das elektrische Stimulationssignal (3), welches von der mindestens einen Elektrode (5a, 5b) übertragen wird, den anterosuperioren Teil des Warzenfortsatzes (101) des Patienten (100) erreicht,
- Erzeugungsmittel (2), welche mit der mindestens einen Elektrode (5a, 5b) verbunden sind, wobei die Erzeugungsmittel (2) dafür ausgebildet sind, der mindestens einen Elektrode (5a, 5b) das elektrische Stimulationssignal (3) bereitzustellten,
**dadurch gekennzeichnet, dass** das elektrische Stimulationssignal (3) eine Hauptfrequenzkomponente größer als 200 kHz und kleiner als 600 kHz aufweist.

2. System nach dem vorhergehenden Anspruch, **dadurch gekennzeichnet, dass** das elektrische Stimulationssignal (3) eine Hauptfrequenzkomponente von zwischen 450 kHz und 540 kHz aufweist.

3. System nach dem vorhergehenden Anspruch, **dadurch gekennzeichnet, dass** das elektrische Stimulationssignal (3) eine Hauptfrequenzkomponente von 495 kHz aufweist.

4. System nach einem der vorhergehenden Ansprüche, **dadurch gekennzeichnet, dass** es eine erste Elektrode (5a) und eine zweite Elektrode (5b) umfasst, welcher auf dem Träger (6) montiert sind, welche so ausgebildet sind, dass, wenn die Elektroden (5a, 5b) auf dem Patienten (100) platziert werden, sie auf dem anterosuperioren Teil des Warzenfortsatzes (101) des Patienten (100) angeordnet werden.

5. System nach dem vorhergehenden Anspruch, **dadurch gekennzeichnet, dass**, in der Richtung des kleinsten Abstands zwischen der ersten Elektrode (5a) und der zweiten Elektrode (5b), der Trennabstand (d1) zwischen der ersten Elektrode (5a) und der zweiten Elektrode (5b) kleiner als der Abstand zwischen den Enden (d2) der ersten Elektrode (5a) und der Abstand zwischen den Enden (d2) der zweiten Elektrode (5b) ist.

6. System nach einem der Ansprüche 4 bis 5, **dadurch gekennzeichnet, dass** die erste Elektrode (5a) und die zweite Elektrode (5b) gleich sind.

7. System nach einem der Ansprüche 4 bis 6, **dadurch gekennzeichnet, dass** die erste Elektrode (5a) und die zweite Elektrode (5b) symmetrisch auf dem Träger (6) angeordnet sind.

8. System nach einem der Ansprüche 4 bis 7, **dadurch gekennzeichnet, dass** die erste Elektrode (5a) und die zweite Elektrode (5b) bogenförmig sind.

9. System nach einem der Ansprüche 4 bis 8, **dadurch gekennzeichnet, dass** die erste Elektrode (5a) und die zweite Elektrode (5b) parallel zueinander sind.

10. System nach einem der vorhergehenden Ansprüche, **dadurch gekennzeichnet, dass** der Träger (6) eine Hörmuschel (10) umfasst, deren Querschnitt (C) einen Hufeisenbogen bestimmt, wobei die Hörmuschel (10) dazu angepasst ist, derart platziert zu werden, dass sie die Ohrmuschel (103) des Patienten (100) deckt und umgibt, wobei die mindestens eine Elektrode (5a, 5b) an einem Ende der Hörmuschel (10) angeordnet ist.

11. System nach einem der vorhergehenden Ansprüche, **dadurch gekennzeichnet, dass** sie auch mit schallabstrahlenden Mitteln (9) versehen ist.

12. System nach dem vorhergehenden Anspruch, **dadurch gekennzeichnet, dass** die schallabstrahlenden Mittel (9) dazu angepasst sind, Schall mit einem Sperrfrequenzband abzustrahlen.

13. System nach dem vorhergehenden Anspruch, **dadurch gekennzeichnet, dass** es Verarbeitungsmittel (11) umfasst, welche dazu angepasst sind, ein Frequenzband eines Audiosignals (12) zu sperren.

14. System nach einem der vorhergehenden Ansprüche, **dadurch gekennzeichnet, dass** der Träger (6) Kopfhörer (7) sind.

## Revendications

1. Système comprenant:
- un dispositif (1) pour traiter des troubles de l'oreille interne tels que l'acouphène ou la perte auditive chez un patient (100) comprenant au moins une électrode (5a, 5b) adaptée pour transmettre un signal de stimulation électrique (3) au patient (100), dans lequel l'au moins une électrode (5a, 5b) est montée sur un support (6) configurée de telle sorte que, lorsqu'elle est placée sur le patient (100), l'au moins une électrode (5a, 5b) est disposée sur la partie antérosupérieure du processus mastoïde (101) du patient (100) de telle sorte que le signal de stimulation électrique (3) transmis à partir de l'au moins une électrode (5a, 5b) atteint la partie antérosupérieure du processus mastoïde (101) du patient (100), et
- des moyens de génération (2) reliés à l'au moins une électrode (5a, 5b), les moyens de génération (2) étant configurés pour fournir le signal de stimulation électrique (3) à l'au moins une électrode (5a, 5b),
**caractérisé en ce que** le signal de stimulation électrique (3) présente une composante de fréquence principale supérieure à 200 kHz et inférieure à 600 kHz.

2. Système selon la revendication précédente, **caractérisé en ce que** le signal de stimulation électrique (3) présente une composante de fréquence principale comprise entre 450 kHz et 540 kHz.

3. Système selon la revendication précédente, **caractérisé en ce que** le signal de stimulation électrique (3) présente une composante de fréquence principale de 495 kHz.

4. Système selon l'une quelconque des revendications précédentes, **caractérisé en ce qu'**il comprend une première électrode (5a) et une deuxième électrode (5b) montée sur le support (6) configurée de telle sorte que, lorsque les électrodes (5a, 5b) sont placées sur le patient (100), elles sont disposées sur la partie antérosupérieure du processus mastoïde (101) du patient (100).

5. Système selon la revendication précédente, **caractérisé en ce que**, dans la direction de la distance la plus petite entre la première électrode (5a) et la deuxième électrode (5b), la distance de séparation (d1) entre la première électrode (5a) et la deuxième électrode (5b) est inférieure à la distance entre les extrémités (d2) de la première électrode (5a) et la distance entre les extrémités (d2) de la deuxième électrode (5b).

6. Système selon l'une quelconque des revendications 4 à 5, **caractérisé en ce que** la première électrode (5a) et la deuxième électrode (5b) sont égales.

7. Système selon l'une quelconque des revendications 4 à 6, **caractérisé en ce que** la première électrode (5a) et la deuxième électrode (5b) sont disposées symétriquement sur le support (6).

8. Système selon l'une quelconque des revendications 4 à 7, **caractérisé en ce que** la première électrode (5a) et la deuxième électrode (5b) sont arquées.

9. Système selon l'une quelconque des revendications 4 à 8, **caractérisé en ce que** la première électrode (5a) et la deuxième électrode (5b) sont parallèles l'une à l'autre.

10. Système selon l'une quelconque des revendications précédentes, **caractérisé en ce que** le support (6) comprend une oreillette (10) dont la section transversale (C) détermine un arc à fer de cheval, l'oreillette (10) étant adaptée pour être placée en recouvrant et en entourant le pavillon de l'oreille (103) du patient (100), l'au moins une électrode (5a, 5b) étant disposée dans une extrémité de l'oreillette (10).

11. Système selon l'une quelconque des revendications précédentes, **caractérisé en ce qu'**il est également pourvu de moyens d'émission sonore (9).

12. Système selon la revendication précédente, **caractérisé en ce que** les moyens d'émission sonore (9) sont adaptés pour émettre du son avec une bande de fréquence d'arrêt.

13. Système selon la revendication précédente, **caractérisé en ce qu'**il comprend des moyens de traitement (11) adaptés pour arrêter une bande de fréquence dans un signal d'audio (12).

14. Système selon l'une quelconque des revendications précédentes, **caractérisé en ce que** le support (6) est un casque (7).
